# EUROPÄISCHE PATENTSCHRIFT

(11) **EP 0 997 118 B1**
(45) Veröffentlichungstag und Bekanntmachung des Hinweises auf die Patenterteilung: **21.11.2001**
(21) Anmeldenummer: 98120600.6
(22) Anmeldetag: 30.10.1998
(51) Int. Cl.: A61F 2/60, A61F 2/68

(54) **Beinprothese**
Leg prosthesis
Prothèse de jambe

(43) Veröffentlichungstag der Anmeldung: 03.05.2000
(73) Patentinhaber: SCHÜTT & GRUNDEI ORTHOPÄDIETECHNIK GmbH, D-23556 Lübeck (DE)
(72) Erfinder: Grundei, Hans, Dr., D-23558 Lübeck (DE)
(74) Vertreter: Fuchs Mehler Weiss & Fritzsche

(56) Entgegenhaltungen:
- EP-A- 0 358 056
- DE-A- 19 627 994
- US-A- 4 143 426
- US-A- 4 158 895
- US-A- 4 776 852

## Beschreibung

Die Erfindung betrifft eine Beinprothese, die an einen Oberschenkelstumpf nach Amputation zu adaptieren ist.

Grundsätzlich unterscheidet man zwischen passiven und funktionellen oder aktiven Prothesen. Mit den letzteren können Bewegungen ausgeführt werden, welche den natürlichen Bewegungsablauf des gesunden Beines nachempfinden.

Theoretisch denkbar wäre hierfür der Einsatz von myoelektrischen Steuerungen. Bei diesen myoelektrischen Steuerungen werden Myosignale der innervierten Muskulatur transkutan abgeleitet, verstärkt und zur analogen oder digitalen Steuerung von Elektromotoren verwendet, die dann entsprechende Prothesenbewegungen ausführen könnten. Allerdings ist der energetische Aufwand bei einer Beinprothese so hoch, daß die Energiedichte bekannter Akkumulatoren nicht ausreichen würde, um einen elektrischen Antriebsmotor für das Kniegelenk für eine akzeptable Zeitdauer mit elektrischem Strom versorgen zu können.

Bei sogenannten Eigenkraftprothesen werden über Bandagenzüge Bewegungen entfernter Körperpartien zur Durcbführung der Prothesenfunktion genutzt. So kann etwa der Vorbringer der Schulter der nichtamputierten Seite zur Öffnung der Prothesenhand genutzt werden. Allerdings sind bislang keine Ansätze bekannt geworden, eine Beinprothese als Eigenkraftprothese auszulegen.

Aus der DE-A-196 27 994 ist ein Oberschenkel-Prothesensystem mit kontrollierbaren Bewegungen nach Ganter bekannt. Darin wird eine Beinprothese beschrieben, bei der von der Muskulatur eines Oberschenkelstumpfes erzeugte Streck- und Beugekräfte direkt in die Prothese eingeleitet werden, d.h., der Patient kann gewillkürt aufgrund körpereigener Kräfte die künstliche Prothese bewegen. Teil des Prothesensystems ist ein sogenanntes Gewichtsaufnahmelager, dessen Ende als Implantat mit dem Oberschenkelknochen verbunden ist. Die kugelförmige Ausbildung soll eine Vergrößerung der Fläche bewirken, über welche die Ablastung des Gewichtes des Patienten erfolgt und somit den Druck innerhalb des Gewebes verringert. Es zeigt sich jedoch, daß diese Versorgung auf längere Zeit unverträglich ist, da die den Oberschenkelstumpf überspannende Haut mit der Zeit verledert und das Gewebe zwischen Haut und Gewichtsaufnahmelager unerträglichen Belastungen ausgesetzt ist.

Aus der US-A-4 143 426 ist ein implantierbarer Adapter bekannt, der eine poröse Oberfläche aufweist, in die Knochenmaterial einwachsen soll. Dieser Adapter ist Teil einer Eigenkraftprothese, bei der Kräfte über ein künstliches Band zu einem Gelenk weitergeleitet werden. Die erzeugten Kräfte stammen von den verbliebenen Muskeln des Trägers des Adapters selbst.

Vor diesem Hintergrund ist es die Aufgabe der vorliegenden Erfindung, einen Vorschlag für eine verträgliche Beinprothese in Form einer Eigenkraftprothese zu unterbreiten.

Erfindungsgemäß wird die Aufgabe gelöst durch eine Beinprothese mit den Merkmalen des Anspruchs 1. Vorteilhafte Weiterbildungen und Ausführungsformen sind in den Unteransprüchen angegeben.

Demgemäß besteht die Beinprothese aus einem Adapter für ein Kniegelenk, dem Kniegelenk selbst und einer daran angekoppelten Unterschenkelprothese, wobei der Adapter mit einem proximalen Stielteil, welches zumindest teilweise mit einer offenmaschigen, dreidimensionalen Raumnetzstruktur bedeckt ist, in den Röhrenknochenstumpf des Oberschenkelstumpfes implantierbar ist und an seinem distalen Ende mit einer Kopplungseinrichtung für das Kniegelenk versehen ist. An ein Unterteil des Kniegelenks ist ein Zügel mit einem oberschenkelstumpfseitig angebrachten Verbindungselement in Form eines Steigbügels zur Anbindung an die Streckmuskulatur des Oberschenkels angekoppelt, in den Streckkräfte zur Streckung des Kniegelenks eingeleitet werden.

Die Streckkräfte werden vom Patienten selbst in der Streckmuskulatur wie beispielsweise im Musculus quadriceps erzeugt. Die Anbindung des kraftübertragenden Elementes an die Muskulatur am Amputationsstumpf erfolgt nach der sogenannten Myokineplastik nach Sauerbruch.

Hierbei wird in der Restmuskulatur im Amputationsstumpf ein Muskelkanal ausgebildet. Durch Einstülpung eines Hautlappens gelingt es, einen verschieblichen Muskelkanal so anzulegen, daß beispielsweise ein Elfenbein- oder Glasstift in ihm geführt werden kann. Der Patient ist in der Lage, durch Innervierung der Stumpfmuskulatur, d.h. des Musculus quadriceps, entsprechende Kräfte aufzubringen und über den Muskelkanal und einem entsprechenden Verbindungselement in das kraftübertragende Element einzuleiten und das künstliche Kniegelenk entsprechend zu strecken. Hierzu dient das oberschenkelstumpfseitig angebrachte Verbindungselement in Form eines Steigbügels.

Der erwähnte Zügel als kraftübertragendes Element ersetzt im wesentlichen das natürliche Patellaband und den Patellaansatz bis hin zur Tuberositas tibiae im gesunden Bein.

Der Adapter für das Kniegelenk ist mit einem proximalen Stielteil in den Röhrenknochenstumpf des Oberschenkelstumpfes setzbar, wobei das Stielteil zumindest teilweise mit einer offenmaschigen dreidimensionalen Raumnetzstruktur bedeckt ist und an seinem distalen Ende mit einer Kopplungseinrichtung für das Kniegelenk versehen ist.

Die offenmaschige, dreidimensionale Raumnetzstruktur, die auch als interkonnektierend bezeichnet wird, ermöglicht ein Ein-, Durch-, Hinter- und Umwachsen von natürlichem Knochenmaterial während der Einheilphase, so daß der Stielteil nach relativ kurzer Dauer jedenfalls im Hinblick auf den Substratfluß im Röhrenknochen integriert wird und eine extrem stabile Sekundärfixation gewährleistet wird.

Durch diese Ausbildung ist ein hinreichend fester Sitz des Adapters am bzw. im Oberschenkelstumpf gewährleistet, der auch den hohen, in das kraftübertragende Element eingeleiteten Kräften bis zu 7845 N (800 kp) standhalten kann.

Eine bislang unbekannt natürlicher Bewegungsablauf ist durch die erfindungsgemäße Beinprothese möglich aufgrund der Anbindung der Restmuskulatur im Oberschenkelstumpf direkt an die am Kniegelenk angekoppelte Unterschenkelprothese.

Aber auch das Kniegelenk ist gemäß einer bevorzugten Ausführungsform in spezieller Weise ausgebildet und an die erfindungsgemäße Beinprothese adaptiert. So ist vorgesehen, daß das Kniegelenk ein Oberteil, das mit dem Adapter verbindbar ist, und ein gelenkig mit dem Oberteil verbundenes Unterteil aufweist, das mit der Unterschenkelprothese verbindbar ist, wobei sich der obere Teil mit Kufen auf Gleitflächen des Unterteils abstützt und die Kufen durch Federwirkung gegen die Gleitflächen gehalten werden und wobei der Oberteil mit den Kufen auf den Gleitflächen bis zum Erreichen einer Strecklage um einen Schwenkpunkt schwenkbar ist, wobei die vertikale Hauptachse des Oberteils gegenüber der vertikalen Hauptachse des Unterteils nach vorne versetzt ist, die Kufen in Seitenansicht im wesentlichen Anlageflächen aufweisen, die von vorne nach hinten gesehen um den Schwenkpunkt stetig kleiner werdende Krümmungsradien einnehmen und die auf den im wesentlichen flach ausgebildeten Gleitflächen des Unterteils aufliegen, und wobei sich das Oberteil gegenüber dem Unterteil bei zunehmender Beugung des Gelenks nach hinten verlagert.

Nähere Einzelheiten zu dieser Ausbildung können der EP 0 358 056 B1 entnommen werden.

In besonders geeigneter Weise eignet sich dieses Kniegelenk für den Einsatz im Rahmen der vorliegenden Erfindung:

Die spezielle Ausbildung der Polkurve der Kufen des Oberteils mit den von vorne nach hinten gesehen stetig kleiner werdenden Krümmungsradien ermöglicht es, die natürlichen Kräfteverhältnisse im Knie nachzuempfinden. So ist die von dem kraftübertragenden Element übertragene Kraft in der Streckstellung des Knies am größten und in der Beugstellung am kleinsten. Dies entsprechend den natürlichen Verhältnissen.

Bei einer anders als angegeben ausgebildeten Polkurve, beispielsweise bei einer kreisförmigen Polkurve, wäre der physiologische Bewegungsablauf des Knies gar nicht möglich.

Das aus der benannten Druckschrift bekannte Kniegelenk ist in besonders vorteilhafter Weise an den Einsatz im Rahmen der vorliegenden Erfindung adaptiert, wenn der Unterteil des Kniegelenks einen Rahmen aufweist, der eine die Gleitflächen aufweisenden Träger abstützt, deren Oberteil durch Achszapfen mit seitlich am Rahmen geführten Laschen verbunden ist, die durch eine Langlöcher durchgreifende Strebe starr verbunden sind, und wobei die Strebe durch eine Feder gegenüber dem Rahmen abgefedert ist und über die Laschen die Kufen gegen die Gleitflächen des Unterteils hält, wobei das erwähnte kraftübertragende Element am Rahmen des Unterteils angeschlagen ist. Die vom Musculus quadriceps erzeugte Kraft wird also direkt vom Oberschenkelstumpf in den Unterteil des Kniegelenks eingeleitet unter Überspannung des Kniegelenks selbst. Dies ist eine exakte Nachbildung der Krafteinleitung durch das Patellaband bzw. Patellaansatz im gesunden Bein.

Die Erfindung wird anhand eines Ausführungsbeispiels gemäß den Zeichnungsfiguren beispielhaft näher erläutert.

Hierbei zeigt:
- Figur 1: eine Seitenansicht der Beinprothese,
- Figur 2: die schematische Ansicht nach operativem Einsatz des Adapters in einen Oberschenkelstumpf, und
- Figur 3: schematisch die Lage des kraftübertragenden Elements nach Anlage der Prothese an einen Oberschenkelstumpf.

Nachfolgend sind gleiche Teile mit denselben Bezugszeichen versehen.

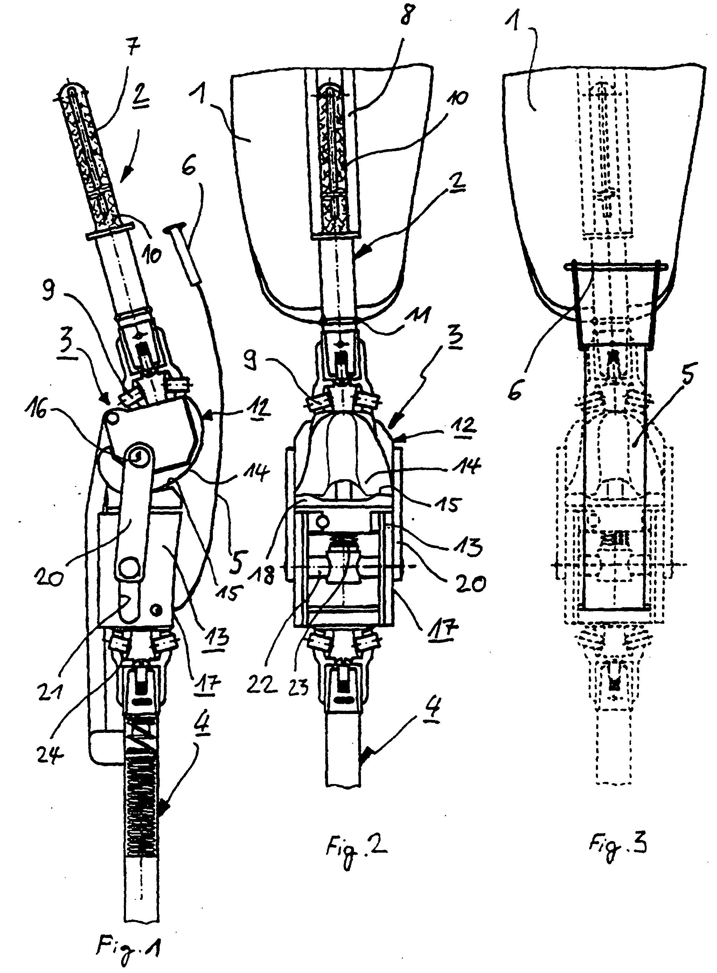

Figur 1 zeigt eine schematische Seitenansicht der kompletten zusammengebauten Beinprothese. Diese weist einen Adapter 2 auf, der mit dem Oberschenkelstumpf 1 (Figur 2) verbunden wird. An den Adapter 2 ist über eine Kopplungseinrichtung 9 ein Kniegelenk 3 angekoppelt. Das Kniegelenk 3 ist über eine weitere Kopplungseinrichtung 24 mit einer Unterschenkelprothese 4 verbunden.

Das Kniegelenk 3 weist ein Oberteil 12 und ein Unterteil 13 auf. Das Oberteil 12 stützt sich über Kufen 14 auf Gleitflächen 15 des Unterteils 13 ab. Bei der Bewegung vom Streckzustand zum Beugezustand schwenkt das Oberteil 12 um einen Schwenkpunkt 16 herum.

Die Kufen 14 sind so ausgebildet und bilden eine solche Polkurve, daß sie in Seitenansicht Anlageflächen aufweisen, die von vorne nach hinten gesehen um den Schwenkpunkt 16 stetig kleiner werdende Krümmungsradien einnehmen. Diese Ausbildung ermöglicht die physiologische Nachbildung der Kräfteverteilung im natürlichen Knie in der Prothese, nämlich dahingehend, daß die größten Kräfte aufgebracht werden müssen, um das Knie in Strecklage zu bringen, wohingegen die geringsten Kräfte notwendig sind in der Beugelage.

Das Unterteil 13 weist einen Rahmen 17 auf, in welchen lateral und medial Langlöcher 21 vorhanden sind. Durch die Langlöcher 21 hindurch greift eine Strebe 22, die seitlich am Rahmen 17 geführte Laschen 20 starr miteinander verbindet. Hierbei ist die Strebe 22 durch eine Feder 23 gegenüber dem Rahmen 17 abgefedert.

Das kraftübertragende Element 5 ist in diesem Ausführungsbeispiel am Rahmen 17 des Unterteils 13 des Kniegelenks 3 angeschlagen. Sein anderes Ende ist als steigbügelartiges Verbindungselement 6 ausgebildet.

Der Adapter 2 wird mit seinem proximalen Stielteil 7, welches mit einer offenmaschigen, dreidimensionalen Raumnetzstruktur 10 bedeckt ist, in den Röhrenknochenstumpf 8 implantiert (vgl. Figur 2).

An der Austritts- oder Durchtrittsstelle des Adapters 2 aus dem Oberschenkelstumpf 1 sitzt ein Abdichtteil 11, das die Durchbruchstelle im Gliedmaßenstumpf schützt und dazu beiträgt, daß sich die Durchbruchsstelle leicht aseptisch halten lassen kann.

Der Rahmen 17 des Unterteils 13 des Kniegelenks 3 stützt im übrigen einen die GleitBächen 15 aufweisenden Träger 18 ab.

Figur 3 ist eine schematische Ansicht von frontal, wobei die Teile aus Figur 2 der Übersicht halber strichliert dargestellt sind. Es ist deutlich veranschaulicht, daß das kraftübertragende Element 5 vorliegend als Zügelband ausgebildet ist, wobei das steigbügelartige Verbindungselement 6 in der eingangs erwähnten Art und Weise an die Streckmuskulatur, bspw. an den Musculus quadriceps nach der erwähnten Myokineplastik nach Sauerbruch angebunden ist.

Das Zügelband kann aus geeignetem Material, wie Kunstfaser, Kunstfasergurte oder auch einem widerstandsfähigen Gummi bestehen. Das Material muß in jedem Falle in der Lage sein, die relativ hohen auftretenden Kräfte bei jeder Streckbewegung zu übertragen. Diese liegen in einem Bereich bis zu 7845 N (800 kp).

## Patentansprüche

1. Beinprothese zur Adaption an einen Oberschenkelstumpf (1), bestehend aus einem Adapter (2) für ein Kniegelenk (3), einem daran befestigten Kniegelenk (3) und einer am Kniegelenk (3) angekoppelten Unterschenkelprothese (4), wobei der Adapter (2) mit einem proximalen Stielteil (7), welches zumindest teilweise mit einer offenmaschigen, dreidimensionalen Raumnetzstruktur (10) bedeckt ist, in den Röhrenknochenstumpf (8) des Oberschenkelstumpfes (1) implantierbar ist und an seinem distalen Ende mit einer Kopplungseinrichtung (9) für das Kniegelenk (3) versehen ist, wobei an dem Unterteil des Kniegelenks ein Zügel mit einem oberschenkelstumpfseitig angebrachten Verbindungselement (6) in Form eines Steigbügels zur Anbindung an die Streckmuskulatur des Oberschenkels angekoppelt ist, in den Streckkräfte zur Streckung des Kniegelenks (3) eingeleitet werden.

2. Beinprothese nach Anspruch 1, bei dem an der Durchtrittsstelle des Adapters (2) aus dem Körper des Patienten ein Abdichtteil (11) sitzt.

3. Beinprothese nach Anspruch 1 oder 2, bei dem das Kniegelenk (3) ein Oberteil (12), das mit dem Adapter (2) verbindbar ist, und ein gelenkig mit dem Oberteil (12) verbundenes Unterteil (13) aufweist, das mit der Unterschenkelprothese (4) verbindbar ist, wobei sich der obere Teil (12) mit Kufen (14) auf Gleitflächen (15) des Unterteils (13) abstützt und die Kufen (14) durch Federwirkung gegen die Gleitflächen (15) gehalten werden und wobei der Oberteil (12) mit den Kufen (14) auf den Gleitflächen (15) bis zum Erreichen einer Strecklage um einen Schwenkpunkt (16) schwenkbar ist, wobei die vertikale Hauptachse des Oberteils (12) gegenüber der vertikalen Hauptachse des Unterteils (13) nach vorne versetzt ist, die Kufen (14) in Seitenansicht Anlageflächen aufweisen, die von vorne nach hinten gesehen um den Schwenkpunkt (16) stetig kleiner werdende Krümmungsradien einnehmen und die auf den im wesentlichen flach ausgebildeten Gleitflächen (15) des Unterteils (13) aufliegen, und wobei sich das Oberteil (12) gegenüber dem Unterteil (13) bei zunehmender Beugung des Gelenks nach hinten verlagert.

4. Beinprothese nach Anspruch 3, bei der der Unterteil (13) des Kniegelenks (3) einen Rahmen (17) aufweist, der einen die Gleitflächen (15) aufweisenden Träger (18) abstützt, der Oberteil (12) durch Achszapfen mit seitlich am Rahmen (17) geführten Laschen (20) verbunden ist; die durch eine Langlöcher (21) durchgreifende Strebe (22) starr verbunden sind, und wobei die Strebe (22) durch eine Feder (23) gegenüber dem Rahmen (17) abgefedert ist und über die Laschen (20) die Kufen (14) gegen die Gleitflächen (15) des Unterteils (13) hält, wobei das kraftübertragende Element (5) am Rahmen (17) des Unterteils (13) angeschlagen ist.

## Claims

1. Leg prosthesis for adaptation to a thigh stump (1), comprising an adapter (2) for a knee joint (3), a knee joint (3) attached thereto and a lower leg prosthesis (4) coupled to the knee joint (3), wherein the adapter (2) having a proximal stem part (7), which is covered at least partly by an open-meshed, three-dimensional spatial network structure (10), can be implanted in the tubular bone stump (8) of the thigh stump (1) and is provided at its distal end with a coupling device (9) for the knee joint (3), wherein a bridle having a connecting element (6) attached on the thigh stump side in the form of a stirrup for binding to the extensor musculature of the thigh is coupled to the lower part of the knee joint, into which thigh extensor forces for extending the knee joint (3) are introduced.

2. Leg prosthesis according to claim 1, in which a sealing part (11) sits at the passage point of the adapter (2) from the body of the patient.

3. Leg prosthesis according to claim 1 or 2, in which the knee joint (3) has an upper part (12), which can be connected to the adapter (2), and a lower part (13) connected flexibly with the upper part (12) and which can be connected with the lower leg prosthesis (4), wherein the upper part (12) is supported by skids (14) on sliding surfaces (15) of the lower part (13) and the skids (14) are held by spring action against the sliding surfaces (15), and wherein the upper part (12) can be pivoted about a pivoting point (16) by the skids (14) on the sliding surfaces (15) until they reach an extended position, wherein the vertical main axis of the upper part (12) is offset forwards with respect to the vertical main axis of the lower part (13), the skids (14) in side view have bearing surfaces, which, seen from the front to the rear, have continuously diminishing radii of curvature about the pivoting point (16) and which rest on the essentially flat-design sliding surfaces (15) of the lower part (13), and wherein the upper part (12) is displaced rearwardly with respect to the lower part (13) with increasing bending of the joint.

4. Leg prosthesis according to claim 3, in which the lower part (13) of the knee joint (3) has a frame (17) which supports a carrier (18) having the sliding surfaces (15), the upper part (12) is connected by axial journals to brackets (20) guided laterally on the frame (17), the brackets (20) being connected rigidly by brace (22) engaging through an elongated hole (21), and wherein the brace (22) is cushioned by a spring (23) with respect to the frame (17) and holds the skids (14) against the sliding surfaces (15) of the lower part (13) via the brackets (20), wherein the force-transmitting element (5) is attached to the frame (17) of the lower part (13).

## Revendications

1. Prothèse de jambe pour une adaptation à un moignon de cuisse (1) se composant d'un adaptateur (2) pour une articulation de genou (3), d'une articulation de genou (3) qui y est accouplée et d'une prothèse de jambe (4) accouplée à l'articulation de genou (3), l'adaptateur (2) pouvant être implanté avec une partie proximale en forme de manche (7), qui est recouverte au moins partiellement d'une structure réticulaire spatiale tridimensionnelle à mailles ouvertes (10), dans le moignon d'os tubulaire (8) du moignon de cuisse (1), et étant muni à son extrémité distale d'un dispositif d'accouplement (9) pour l'articulation de genou (3), une bride avec un élément de liaison (6) en forme de bride montante fixé du côté du moignon de cuisse, dans laquelle sont introduits des efforts d'étirement pour l'étirement de l'articulation de genou (3), étant accouplée à la partie inférieure de l'articulation de genou pour permettre un raccordement à la musculature d'étirement de la cuisse.

2. Prothèse de jambe selon la revendication 1, **caractérisée en ce que** le point de passage de l'adaptateur (2) hors du corps du patient comprend une pièce d'étanchéité (11).

3. Prothèse de jambe selon la revendication 1 ou 2, **caractérisée en ce que** l'articulation de genou (3) comprend une partie supérieure (12) qui peut être reliée à l'adaptateur (2) et une partie inférieure (13) reliée de manière articulée à la partie supérieure (12) et qui peut être raccordée à la prothèse de jambe (4), la partie supérieure (12) s'appuyant au moyen de patins (14) sur des surfaces de glissement (15) de la partie inférieure (13) et les patins (14) étant maintenus contre les surfaces de glissement (15) par une action de ressort, la partie supérieure (12) avec les patins (14) pouvant pivoter sur les surfaces de glissement (15) autour d'un point de pivotement (16), jusqu'à ce qu'une position d'étirement soit atteinte, l'axe principal vertical de la partie supérieure (12) étant décalé vers l'avant par rapport à l'axe principal vertical de la partie inférieure (13), les patins (14) comprenant en vue latérale sensiblement des surfaces d'appui qui, vu de l'avant vers l'arrière, présentent des rayons de courbure se rétrécissant constamment autour du point de pivotement (16) et qui reposent sur les surfaces de glissement (15) de la partie inférieure (13) dont la forme est essentiellement plate, et la partie supérieure (12) se décalant vers l'arrière par rapport à la partie inférieure (13), lorsque le fléchissement vers l'arrière de l'articulation s'accroît.

4. Prothèse de jambe selon la revendication 3, **caractérisée en ce que** la partie inférieure (13) de l'articulation de genou (3) comprend un cadre (17) qui soutient un support (18) comprenant les surfaces de glissement (15) et **en ce que** la partie supérieure (12) est reliée par des tourillons d'axes à des barrettes (20) disposées latéralement sur le cadre (17) et qui sont reliées de manière rigide par une entretoise (22) traversant des trous oblongs (21), l'entretoise (22) s'appuyant sur le cadre (17) par l'intermédiaire d'un ressort (23) en maintenant les patins (14) contre les surfaces de glissement (15) de la partie inférieure (13) au moyen des barrettes (20) et l'élément de transmission d'efforts (5) étant accroché sur le cadre (17) de la partie inférieure (13).
